# EUROPEAN PATENT SPECIFICATION

(11) **EP 2 836 165 B1**
(45) Date of publication and mention of the grant of the patent: **14.10.2015**
(21) Application number: 13723955.4
(22) Date of filing: 15.03.2013
(51) Int. Cl.: A61F 2/24, A61M 25/04, A61B 19/00

(54) **INTRACORPOREAL IMAGING AID (IMA)**
HILFSVORRICHTUNG FÜR INTRAKORPORALE BILDGEBUNG
AIDE À L'IMAGERIE INTRACORPORELLE (IMA)

(30) Priority: 12.04.2012 US 201261623404 P
(43) Date of publication of application: 18.02.2015
(73) Proprietor: Medivalve Ltd., 20174 Misgav (IL)
(72) Inventor: KLEIN, Assaf, 3885700 Hama'pil (IL); HEFER, Gil, 1790600 Shimshit (IL); GILBOA, Hadar, 3460070 Haifa (IL)
(74) Representative: Hocking, Adrian Niall
(86) International application number: PCT/IB2013/052089
(87) International publication number: WO 2013/153470

(56) References cited:
- WO-A1-2010/119445
- WO-A2-2005/039428
- US-A1- 2007 038 293
- US-A1- 2011 313 283

## Description

### RELATED APPLICATIONS

The present application claims the benefit under 35 U.S.C. 119(e) of U.S. Provisional Application 61/623,404 filed on April 12, 2012.

### TECHNICAL FIELD

Embodiments of the present invention relate to devices for imaging internal features of the body.

### BACKGROUND

The aortic valve is located in the wall of the left ventricle at the entrance of the aorta and operates to prevent blood pumped by the left ventricle into the aorta from flowing back into the heart. The aortic valve may become leaky or blocked, causing a condition known as aortic insufficiency. In such patients, replacement of the aortic valve may be recommended. Transcatheter Aortic Valve Implantation (TAVI) is a procedure in which a patient's defective aortic valve is replaced with a prosthetic valve in a procedure using a catheter, rather than in a procedure involving sternotomy, opening the patient's chest cavity. Advantages of TAVI over sternotomy include reduced risk of medical complication and shorter recovery times.

A difficulty in performing TAVI is ensuring proper placement of the prosthetic valve, in the place of the defective valve. Improper placement of the prosthetic heart valve may not cure the patient's aortic valve complication, and may even cause harm to the patient by blocking proper blood flow and cause additional complications. This difficulty in performing TAVI stems from the fact that heart tissue is difficult to visualize using standard imaging procedures that are typically employed to guide a surgeon in performing the procedure.

Document WO 2010/119445 A1 discloses an apparatus according to the preamble of claim 1.

### SUMMARY

The present ivention concerns an apparatus for determining a location of a surface of an internal organ as claimed in claim 1. Preferred embodiments are recited in the dependent claims.

An aspect of an embodiment of the invention relates to providing apparatus, hereinafter referred to as an intracorporeal imaging aid (IMA), for positioning markers that are readily imaged using a conventional medical imaging modality on a surface of an internal body tissue to aid in identifying and making a location of the surface visible using the imaging modality. The markers may be referred to as tissue markers. An exemplary surface which may be identified using an IMA is the surface of the aortic valve and/or tissue surrounding the aortic valve.

In an embodiment of the invention, an IMA comprises a marker support comprising at least three elastically deformable support filaments each comprising at least one tissue marker, and having distal and proximal ends. Each tissue marker may be located on or in the filament substantially midway between the distal and proximal ends of the filament in a region referred to as an abutment region. The marker support may further comprise a cylindrical distal collar, to which the distal ends of the filaments are connected. The marker support may also comprise a cylindrical proximal collar, to which the proximal ends of the filaments are optionally connected. The marker support has a collapsed state and a deployed state.

In the collapsed state, the distal and proximal ends of each filament are substantially maximally apart and the filaments lie within a relatively small radial distance from the axis of the marker support. In an embodiment of the invention, the radial distance is small enough so that the IMA may be introduced into the body using a catheter deployment system having dimensions adapted for introduction through the cardiovascular system and passage through the aortic valve. The marker support may be positioned on a guidewire while introduced into the body, and deployment may occur while the marker support is located on the guidewire.

The IMA may be brought to the deployed state by moving the distal and proximal ends of the marker support towards each other until they are within a predetermined deployed distance, at which the marker support may be maintained deployed in the stable state. In the deployed state, at initial stages of deployment, the filaments may form loop-like, substantially planar structures whose planes extend along radial directions substantially from the axis of the marker support. Upon further motion of the proximal and distal collars towards each other, the loop-like structures, hereinafter also referred to as "support loops", contort and rotate away from their initial respective radial directions. When the proximal and distal collars are within the predetermined deployment distance, each of the support loops has a proximal end that comprises the abutment region of the loop's filament, and projections of the abutment regions on a plane perpendicular to the axis of the IMA lie along the sides of a polygon, hereinafter referred to as a projection polygon. For an IMA in accordance with an embodiment of the invention comprising not more than three support filaments the projection polygon is a projection triangle. Optionally, the projection triangle is substantially an equilateral triangle. The shape of a support loop when the IMA is in the deployment state may be referred to as a "loop deployment shape".

In an embodiment of the invention, each support filament is processed so that upon bringing the proximal and distal ends of the IMA within the deployment distance of each other, the filament naturally assumes the loop deployment shape. Optionally, processing the filament to assume the loop deployment shape comprises annealing the filament on a suitable mandrel or die that maintains the filament in the loop deployment shape during annealing and/or shape setting by heat treatment. In some embodiments of the invention, the filament is formed from a suitable shape memory or superelastic alloy treated to remember the loop deployment shape.

An IMA may be introduced in the collapsed state into and positioned inside a body using a suitable catheter and guide wire. In an embodiment of the invention, a deployment tube and guide wire may be used to move the distal and proximal ends of the IMA towards one another and bring the IMA from its collapsed state to its deployed state. In the deployed state inside the body the IMA may be moved proximally toward a tissue surface inside the body to be located so that the abutment regions of the support loops and their respective markers abut the tissue. An image of at least three markers acquired using a suitable medical imaging modality may be used to locate the markers and thereby determine orientation of a plane along which the abutted tissue substantially lies.

In an embodiment of the invention an IMA may be used to determine orientation of a planar region of tissue in which the aortic valve of a patient undergoing TAVI is located. Location of the planar region may be determined with relatively small risk of a support loop lodging in a commissure of the aortic valve because the support loops of a deployed IMA lie along sides of a polygon and thereby, in general, transverse to the valve commissures.

According to an embodiment of the invention, the marker support may further comprise a proximal deployment marker at its proximal end (either on a filament or on a collar) and a distal deployment marker at its distal end (either on a filament or on a collar.) The distance between the proximal marker and distal marker may be identified using an imaging modality to determine if the marker support is in a deployed state or in a collapsed state.

According to an embodiment of the invention, relative motion between proximal marker and distal marker may be identified using an imaging modality to determine if the marker support is properly placed against a surface of an internal body tissue.

In the discussion unless otherwise stated, adjectives such as "substantially" and "about" modifying a condition or relationship characteristic of a feature or features of an embodiment of the invention, are understood to mean that the condition or characteristic is defined to within tolerances that are acceptable for operation of the embodiment for an application for which it is intended. Unless otherwise indicated, the word "or" in the specification and claims is considered to be the inclusive "or" rather than the exclusive or, and indicates at least one of, or any combination of items it conjoins.

This summary is provided to introduce a selection of concepts in a simplified form that are further described below in the detailed description. This summary is not intended to identify key features or essential features of the claimed subject matter, nor is it intended to be used to limit the scope of the claimed subject matter.

### BRIEF DESCRIPTION OF FIGURES

Non-limiting examples of embodiments of the invention are described below with reference to figures attached hereto that are listed following this paragraph. Identical structures, elements or parts that appear in more than one figure are generally labeled with a same numeral in all the figures in which they appear. Dimensions of components and features shown in the figures are chosen for convenience and clarity of presentation and are not necessarily shown to scale.
Figs. 1A and 1B show an IMA in closed configuration according to an embodiment of the invention;
Fig. 1C shows an IMA according to an embodiment of the invention in collapsed configuration, in disassembled format;
Fig. 1D shows distal ends of collapsed IMAs according to embodiments of the invention;
Figs. 2A-2I show cross-sectional views of the left ventricle of a patient with an IMA according to embodiments of the invention, at various stages of deployment.

### DETAILED DESCRIPTION

As mentioned above, IMAs according to embodiments of the invention are useful in imaging a patient's aortic valve and in aiding in replacement thereof. IMAs according to embodiments of the invention may be used to aid in medical procedures performed in a minimally invasive manner, obviating the need for large incisions.

In addition to imaging the aortic valve, various other imaging procedures may be performed using IMAs according to embodiments of the invention. For example, cardiovascular structures such as vessels, arteries, veins, branches, occlusions, blockages, chambers and valves may be visualized. Gastrointestinal structures such as the throat, esophagus, stomach, duodenum, intestines, colon and blockages therein may also be visualized. Pulmonary structures such as trachea, bronchi and thorax may also be visualized. Uro-gynecological structures such as the ureter, bladder, cervix, uterus, fallopian tubes and blockages may also be visualized.

In addition to prosthetic valves, other medical devices may be positioned with devices according to embodiments of the invention including but not limited to, stents, catheters, balloon catheters, pace makers, radioactive medicines, therapeutics, cameras, laparoscopes, endoscopes and sterilization devices.

IMAs according to embodiments of the invention may be used to direct laparoscopic or endoscopic surgery or to visualize target anatomical structures with directed energy therapies such as radiation therapy.

The term distal refers to a direction away from the opening in the body along the axis of the guidewire or catheter used to insert the IMA. The term proximal refers to a direction towards the opening in the body along the axis of the guidewire or catheter.

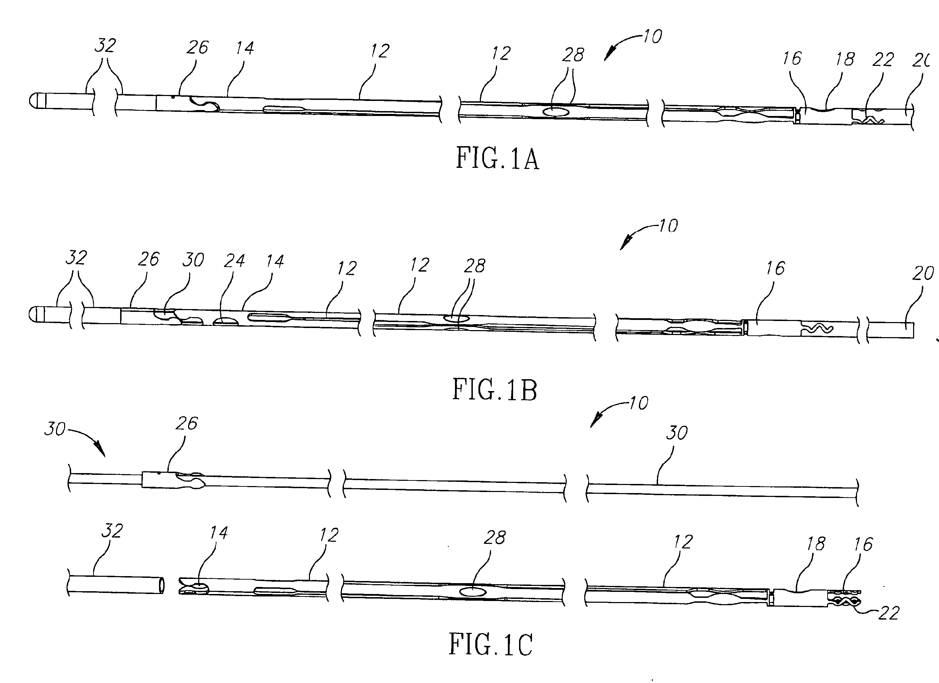

Reference is now made to the figures which illustrate various embodiments of an IMA according to embodiments of the invention, adapted for the performance of TAVI. As shown in Fig. 1A, 1B and 1C, (disassembled view) an IMA 10 is shown in a collapsed position. IMA 10 comprises a marker support 12 having a distal collar 14 and a proximal collar 16. Proximal collar 16 comprises a proximal deployment marker 18. Distal collar 14 comprises a distal deployment marker 24. Marker support 12 comprises tissue markers 28.

Marker support 12 is configured to be movably attached, with an ability to slide along a guidewire 30 (guidewire shown in Figs. 1B and 1C). Marker support 12 is fixedly attached at proximal collar 16 to outer deployment tube 20 via connector 22. Clip connector 26 is fixedly attached to guidewire 30. Distal collar 14 of marker support 12 may be clipped to clip connector 26 as in Fig. 1A or unclipped as in Fig. 1B. The portion of guidewire 30 shown in figures 1A-1C is substantially coincident with the axis of IMA 10.

Guidewire 30 may be coated with envelope 32, at the distal end of guidewire 30.

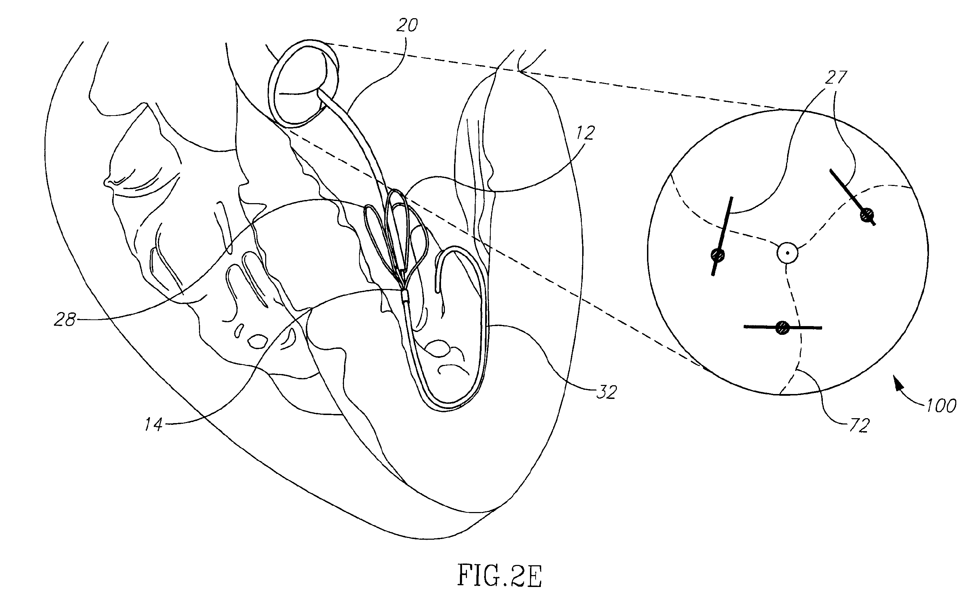

Marker support 12 may be formed from metals such as stainless steel, shape memory alloys, superelastic alloys, titanium alloys, surgical steel, zirconium alloys, niobium alloys, tantalum alloys, polymers or other flexible and medically safe materials. According to an embodiment of the invention, marker support 12 is made from a nickel-titanium alloy. Marker support 12 may be formed, for example by cutting filaments using laser cutting techniques or by using wires optionally attached to collars. Marker support 12 may be shaped in its deployed position (shown below in figures 2E-2H) by shape setting in its deployed position. After being collapsed, marker support 12 may revert to its deployed position upon release of tension between distal and proximal ends of marker support 12 or by pushing proximal end and distal end towards eachother.

According to an embodiment of the invention, marker support 12 is shaped, for example by annealing, in a manner that each filament comprises a single strand deformed to a position capable of forming a loop comprising an abutment member.

Tissue marker 28, distal marker 24 and proximal marker 18 may all be formed from a radio-opaque material, preferably material which is easily discernible from native human tissue when using imaging techniques such as fluoroscopy, computed tomography (CT), ultrasound, magnetic resonance imaging (MRI), positron emission tomography (PET). In an embodiment of the invention, a marker may be formed from stainless steel, ceramic, titanium alloy, tungsten, zirconium, silver, platinum, tantalum or gold.

Guidewire 30 and outer deployment tube 20 may be formed using guidewires and deployment tubes known in the art.

As shown in figures 1A-1C, IMA 10 is in the collapsed state and has a tubular shape. The distal and proximal ends of each filament are substantially maximally apart and the filaments lie within a relatively small radial distance from the axis of the IMA , the axis being parallel and coincident with the guidewire. In an embodiment of the invention, the radial distance is small enough so that the IMA may be introduced into the body using a catheter deployment system having dimensions adapted for introduction through the cardiovascular system and passage through the aortic valve.

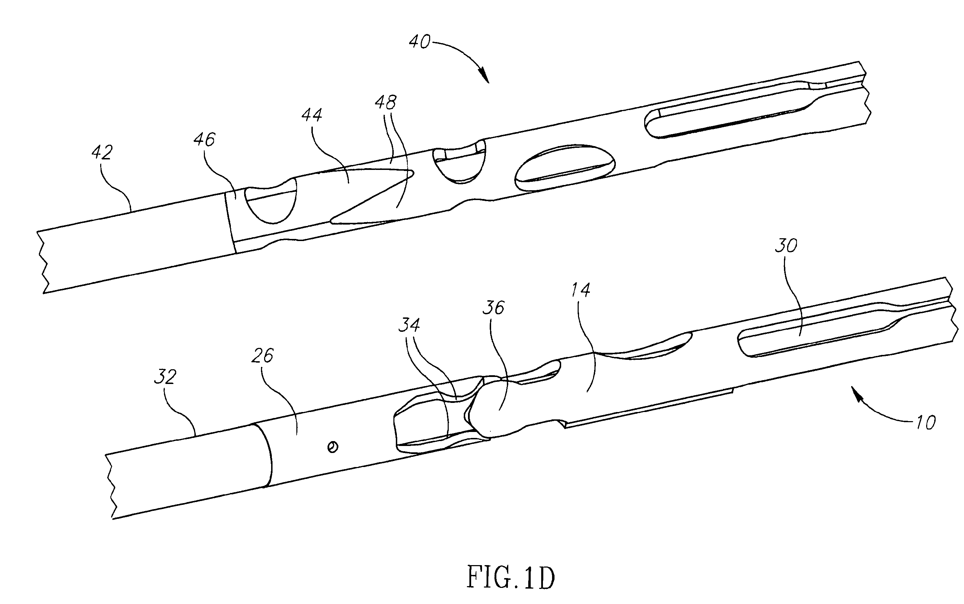

With reference to Fig. 1D, IMA 10 is shown in collapsed state, using an enlarged view of distal collar 14. Distal collar 14 comprises fingers 36. Clip connector 26, which is fixedly attached to guidewire 30, comprises fingers 34. Fig. 1D shows fingers 34 and figure 36 in partially engaged position. In this partially engaged position, rotational movement of distal collar 14 relative to clip connector 26 (and relative to guidewire 30, which is fixedly attached to clip connector 26) is limited due to the partial engagement of fingers 34 and fingers 36. Upon applying significant force on marker support 12 in the distal direction, fingers 36 enter apertures formed between fingers 34, thereby entering engaged position. In engaged position, application of significant force is required to detach clip connector 26 from distal collar 14. For example, the force required to detach clip connector 26 from distal collar 14 is higher than the force required to transform marker support 12 from a deployed state to a collapsed state.

Fig. 1D also shows IMA 40 according to an embodiment of the invention, with an enlarged view of a distal end of a marker support according to an embodiment of the invention. Marker support comprises fingers 48 and clip connector 46 comprises fingers 44. Fig. 1D shows fingers 44 engaged with fingers 46, thereby preventing rotational movement of marker support relative to clip connector 46. Upon movement of marker support 12 in a proximal direction relative to clip connector 46 from the position shown in the figure, fingers 48 will disengage from fingers 44 without significant resistance.

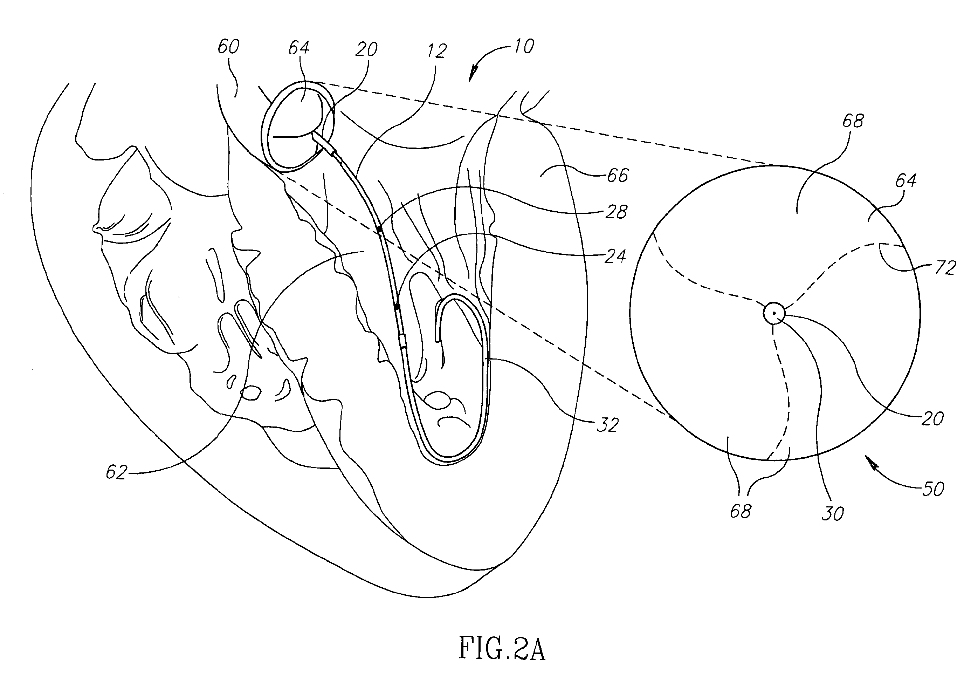

Figures 2A-I show cross sectional views of a patient's left ventricle 62, surrounded by a left ventricle wall 66 at various stages of deployment of an IMA.

Figure 2A shows an IMA 10 positioned in collapsed configuration in the patient's left ventricle. IMA 10 is introduced through an aorta 60 via an aortic valve 64 in need of replacement. IMA 10 may be introduced in a catheter tube (not shown) which is then removed to allow for expansion of marker support 12.

Inset 50 shows a projection of a plane perpendicular to guidewire 30, viewed along the axis of deployment tube 20 and guidewire 30, including aortic valve 64. Aortic valve 64 comprises leaflets 68 which are separated by commissures 72 which separate leaflets 68 when valve 64 is closed.

In an embodiment of the invention, envelope 32 may have a curved tip to allow for positioning in the left ventricle as shown in figure 2A, without puncturing heart tissue. In an embodiment of the invention, envelope 32 may be coated with a protective layer, for example, a coated wire, to prevent puncturing of heart tissue. Positioning of envelope 32 limits further movement of guidewire 30 in the distal direction.

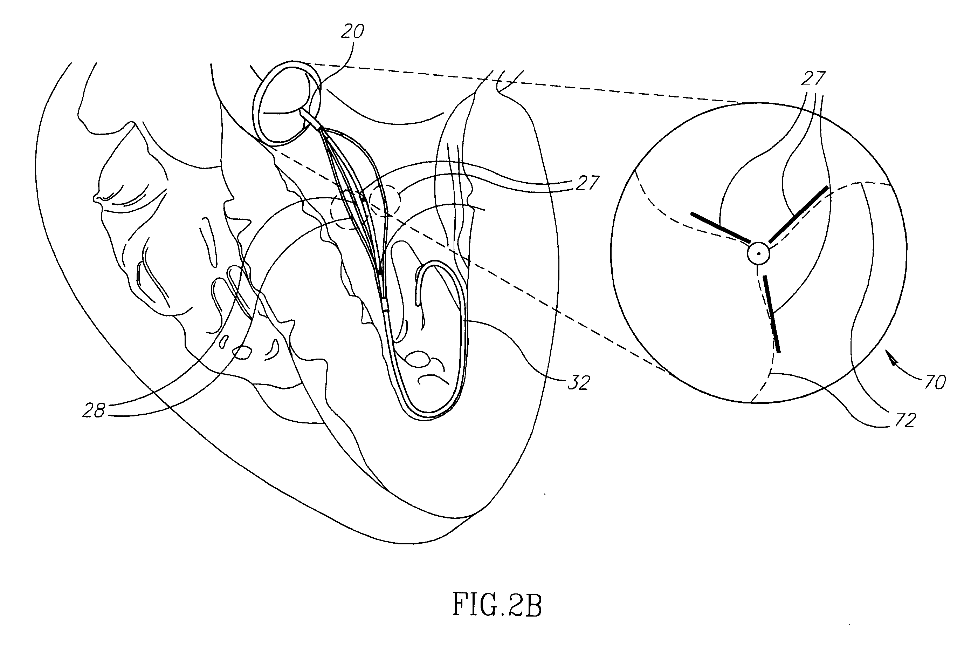

Fig. 2B shows an IMA 10 positioned in the patient's left ventricle, during the initiation of the deployment process. Upon movement of proximal collar 16 and distal collar 14 towards one another by, for example, distal movement of deployment tube 20, marker support 12 is compressed, separating three deployable filaments, which begin to form loop-like structures. Each deployable filament comprises a tissue marker 28 and an abutment region 27.

Inset 70 shows a projection of abutment regions 27 on a plane perpendicular to guidewire 30, viewed along the axis of deployment tube 20 and guidewire 30, including aortic valve 64. Abutment regions 27 are shown extending in a radial direction from guidewire 30, each loop defining a plane extending radially from the axis.

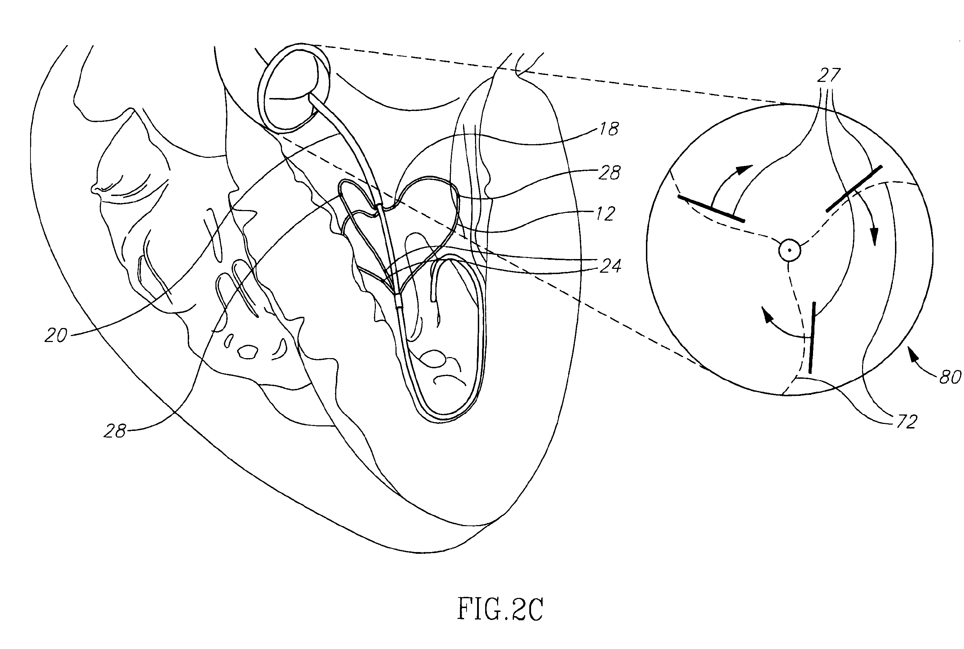

Fig. 2C shows an IMA 10 positioned in the patient's left ventricle, during the deployment process, in a stage following the position shown in Fig. 2B. Upon further movement of proximal collar 16 in the direction of distal collar 14, filaments assume a loop-like configuration each loop defining a plane extending radially from the axis.

Inset 80 shows a projection of abutment regions 27 on a plane perpendicular to guidewire 30, viewed along the axis of deployment tube 20 and guidewire 30, including aortic valve 64. Projection of abutment regions 27 extend in a radial direction from guidewire 30. In such a formation, if marker support 12 is applied to aortic valve 64, abutment regions 27 may enter commissures 72, thereby providing an inaccurate image of location of aortic valve.

Upon further movement of proximal collar 16 and distal collar 14 towards each other, projection of abutment regions 27 begin to move relative to commissures 72 in the clockwise direction as indicated by arrows.

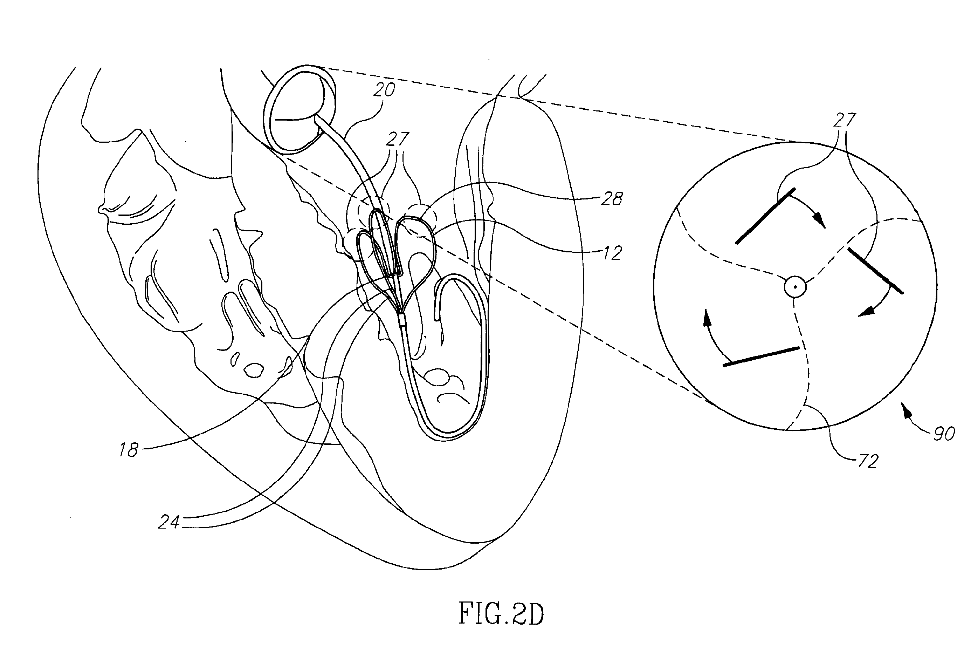

Fig. 2D shows an IMA 10 positioned in the patient's left ventricle, during the deployment process, in a stage following the position shown in Fig. 2C.

Upon further movement of proximal collar 16 and of distal collar 14 toward each other, marker support 12 is further distorted as loops further rotate away from their initial radial directions, and abutment regions 27 move to point in the proximal direction.

Inset 90 shows a projection of abutment regions 27 on a plane perpendicular to guidewire 30, viewed along the axis of deployment tube 20 and guidewire 30, including aortic valve 64. Abutment regions 27 move in the clockwise direction as indicated by arrows.

In an embodiment of the invention, abutment regions 27 are moved in the counterclockwise direction upon distal compression of marker support 12.

As mentioned with reference to Fig. 1D, distal collar 14 of marker support 12 may be partially engaged with clip connector 26, which is fixedly attached to guidewire 30, to prevent rotational movement of distal collar 14 relative to clip connector 26 (and relative to guidewire 30, which is fixedly attached to clip connector 26.) This configuration allows marker support 12 to be aligned so that deployable elements avoid excessive contact with left ventricle wall 66 while deploying, thereby preventing entanglement of marker support 12 with left ventricle wall 66.

Fig. 2E shows an IMA 10 positioned in the patient's left ventricle, during the deployment process, in a stage following the position shown in Fig. 2D.

Upon further movement of proximal collar 16 and distal collar 14 towards each other to a predetermined distance, support loops are rotated so that each loop defines a plane substantially normal to a vector extending radially from the axis.

Inset 90 shows a projection of abutment regions 27 on a plane perpendicular to guidewire 30, viewed along the axis of deployment tube 20 and guidewire 30, including aortic valve 64. Projections of abutment regions on the plane form a polygon, in particular a substantially equilateral triangular shape. In the formation as shown in Inset 100, abutment regions 27 may be placed on aortic valve 64 without the risk of misplacement which may be caused by an abutment region 27 entering a commissure 72.

According to an embodiment of the invention, the projection polygon is a tangential polygon in which all sides of the polygon are tangent to an inscribed circle. According to an embodiment of the invention, the projection polygon is a rectangle, a square, a pentagon, a hexagon or an octagon. According to an embodiment of the invention, the polygon is an equiangular polygon.

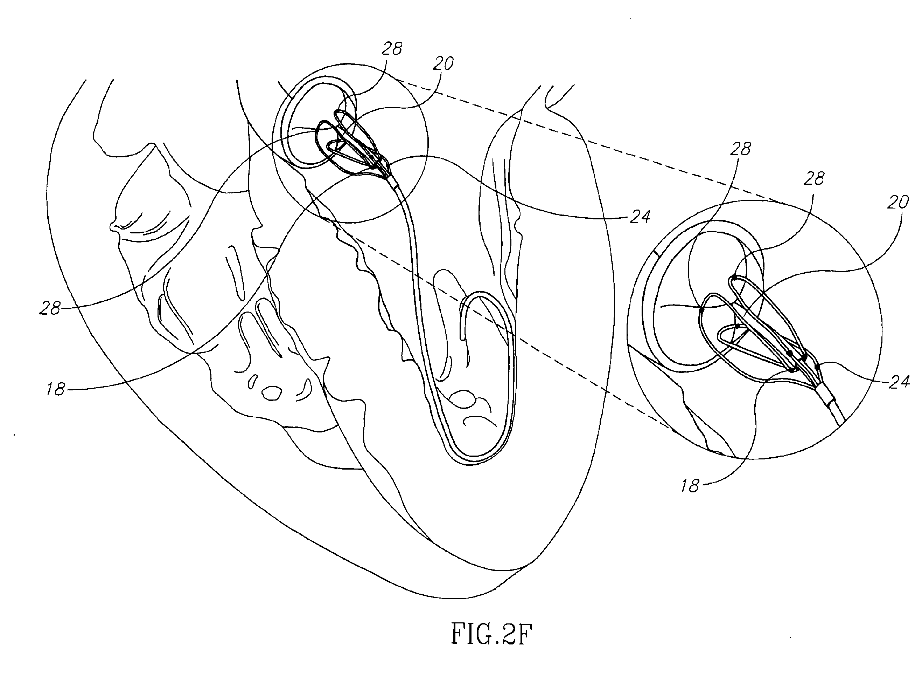

Once marker support 12 has been deployed as in Fig. 2E, it may be positioned against aortic valve as in Fig. 2F. Loops formed by deployable filaments are substantially are pointed in a substantially proximal direction. Marker support 12 may be positioned against aortic valve by moving deployment tube 20 proximally relative to guidewire 30. A medical practitioner may realize that a marker guide is properly positioned, for example, through feeling increased resistance while moving deployment tube 20 proximally when abutment members 27 of marker support 12 abut against aortic valve.

In an embodiment of the invention, proper deployment of marker support 12 may be verified by an imaging modality to determine if distance between the proximal deployment marker 18 and distal deployment marker 24 corresponds to a predetermined distance associated between the markers in deployed state.

Fig. 2F shows abutment region 27 and tissue markers 28 abutted against aortic valve. Proximal deployment marker 18 and distal deployment marker 24 are shown and may be easily visualized relative to each other using imaging techniques. Proper deployment of marker support 12 may be verified by imaging relative motion of proximal deployment marker 18 away from distal deployment marker 24 upon gentle pulling of deployment tube 20 in the proximal direction, thereby slightly compressing marker support 12. Upon distal motion, for example, by release of deployment tube 20, and allowing marker support 12 to spring back in the distal direction, proximal deployment marker 18 moves towards distal deployment marker 24.

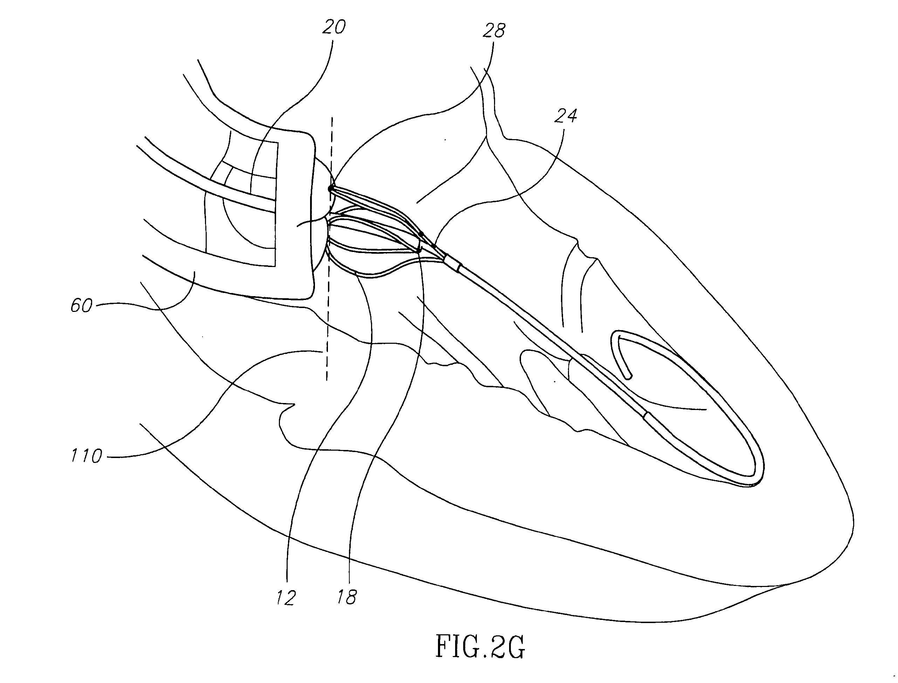

Fig. 2G shows a side view of marker support 12 positioned against aortic valve. A dotted line 110 indicates the plane defined by tissue markers 28 abutted against aortic valve. A prosthetic valve may be introduced, for example, using a guide tube external to deployment tube 20

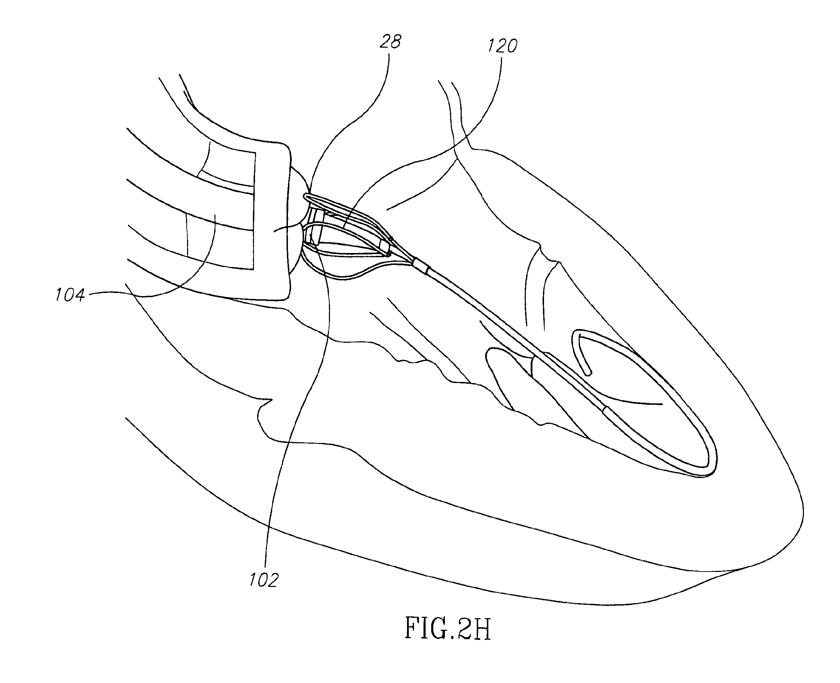

Fig. 2H shows introduction of a collapsed prosthetic valve 104 via valve guide 120, having a valve marking 102. Valve marking 102 may be imaged relative to tissue markers 28 to confirm proper alignment of compressed prosthetic valve 104 relative to a plane defined by tissue markers 28, thereby confirming proper placement of prosthetic valve 104.

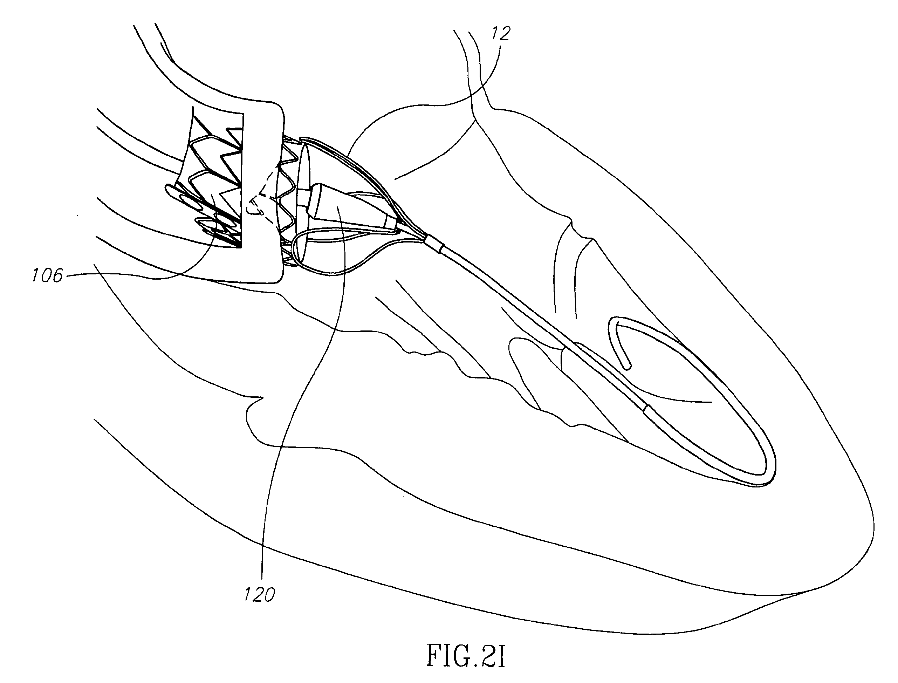

Figure 2I shows expanded prosthetic valve 106 which is expanded, thereby replacing valve 64 upon verification of prosthetic valve location. Valve guide 120 may then be removed from the patient, and the removal of IMA may be initiated.

IMA may be removed by moving deployment tube 20 distally relative to guidewire 30, thereby moving deployed marker support 12 distally. Upon contact of distal collar 14 with clip connector 26, distal force may be applied to deployment tube 20 to clip distal collar 14 to clip connector 26. Upon clipping, deployment tube 20 may be pulled proximally, thereby pulling proximal collar 16 of marker support 12 relative to guidewire 30 thereby closing marker support to a collapsed position as shown in figure 2A. IMA may then be removed by introduction of a catheter external to deployment tube 20 and pulling of guidewire 30 and deployment tube 20 through the catheter.

In an embodiment of the invention, a distal collar may be configured to lock with a clip connector by rotation, for example using matching threading on the distal collar and the clip connector. Alternatively, the distal collar may be configured to lock with a clip connector using a quarter-turn locking mechanism.

In an embodiment of the invention, an IMA may be formed as in Fig. 1A with a modification of an intermediate tube configured to be attached and slidable surrounding the guidewire and beneath the deployment tube. A distal end of a marker support may be fixedly attached to the intermediate tube. A proximal end of a marker support may be fixedly attached to the outer deployment tube. Deployment of the marker support may occur through distal pushing of the outer deployment tube relative to the intermediate tube, or alternatively through proximal pulling of the intermediate tube relative to the outer deployment tube. Closing of the marker support may occur through distal pushing of the intermediate tube relative to the deployment tube, or alternatively by proximal pulling of the deployment tube relative to the intermediate tube. Such a configuration may allow for rapid collapsing and deployment of a marker support without a clip connector affixed to the guidewire.

Embodiments of the invention described in the figures showed IMAs comprising marker supports comprising 3 filaments and 3 loop-like structures. Further embodiments of the invention comprise marker supports having 4, 5, 6, 7 or 8 filaments or 4, 5, 6, 7 or 8 loop-like structures which extend to abut a surface of an internal body tissue.

In an embodiment of the invention, a marker support may be formed by deployable elements which upon deployment of the marker support, are positioned to form a circumferential formation relative to the guidewire.

In an embodiment of the invention, deployable elements may comprise a mesh- like material, comprised of filaments, which is flexible, and tubular in collapsed state. Upon deployment, deployable elements may then extend from to form a concentric formation relative to the guidewire.

In an embodiment of the invention, deployable elements, upon initial deployment, may be substantially extending in a radial direction from the axis. Deployable elements may be rotated to a formation in which projections of the abutment regions on a plane perpendicular to the axis of the marker support each lie along a side of a polygon, for example, by rotation of a deployment tube by a medical practitioner, relative to a guidewire.

Embodiments of the invention described in the figures showed IMAs comprising abutment regions which comprised within them tissue markers. Further embodiments of the invention relate to IMAs comprising a tissue marker located in or on a filament, located a predetermined distance away from an abutment region. A medical practitioner may use an imaging modality to visualize a plane formed by the tissue markers, the plane being located a predetermined distance away from a surface of an internal body tissue. A medical practitioner may then optionally position a valve or device in line with or relative to the imaged plane formed by the tissue markers.

There is further provided in accordance with the present disclosure an apparatus for determining a location of a surface of an internal organ of a body, the apparatus comprising: a marker support having proximal and distal ends, an axis, a collapsed state and a deployed state, the marker support comprising: at least three flexible filaments, which in the collapsed state lie within a relatively small radial distance from the axis of the marker support, and in the deployed state, each filament forms a loop, the loops each configured to abut a surface of an internal body tissue at an abutment region of each filament; and at least three tissue markers, each tissue marker located at an abutment region of a filament; wherein projections of each of the abutment regions on a plane perpendicular to the axis of the marker support each lie along a side of a polygon. Optionally, upon initial transition from the collapsed state to the deployed state, the loops define planes which extend along radial directions from the axis of the marker support. Optionally, the marker support further comprises a proximal collar at a proximal end and a distal collar at a distal end, each filament connected to the proximal collar and distal collar. Optionally, bringing the proximal collar closer to the distal collar transforms the marker support from a collapsed state to a deployed state. Optionally, in the deployed state, the tissue markers are configured to abut a surface of an internal body tissue by movement of the marker support in a proximal direction. Optionally, a tissue marker is located substantially midway between the distal and proximal ends of the filament. Optionally, the marker support is in a collapsed state, the radial diameter is small enough so that the marker support may be introduced into a human body using a guiding catheter system having dimensions adapted for introduction through the cardiovascular system and passage through the aortic valve. Optionally, the apparatus further comprising a guidewire and a deployment tube external to the guidewire. Optionally, the marker support is slidably attached to a guidewire. Optionally, the marker support is fixedly attached to the deployment tube at the proximal end of the marker support. Optionally, the apparatus comprises a connector fixedly attached to the guidewire, the connector configured to engage the distal collar of the marker support upon relative motion of the connector in the direction of the distal collar. Optionally, a protrusion on the connector engages the distal collar of the marker support, preventing rotational movement of the distal collar relative to the connector. Optionally, the connector and distal collar interlock via a clipping mechanism located on the connector and the distal collar. Optionally, the apparatus further comprises an intermediate tube between the guidewire and the deployment tube. Optionally, the distal collar is fixedly attached to the intermediate tube and the proximal collar is fixedly attached to the deployment tube. Optionally, when the marker support is in deployed state, it is shaped so that each filament comprises a single strand deformed to a position capable of forming a loop comprising an abutment region. Optionally, the marker support comprises a distal marker on its distal end and a proximal marker on its proximal end configured to be imaged overlapping or at a predetermined distance from each other when the marker support is in its deployed state. Optionally, the surface of an internal body tissue is a surface of the aortic valve. Optionally, the marker support comprises a superelastic alloy. Optionally, the superelastic alloy is nitinol. Optionally, the polygon is a tangential polygon in which all sides of the polygon are tangent to an inscribed circle. Optionally, the polygon is equiangular. Optionally, the polygon is a rectangle, a square, a pentagon, a hexagon or an octagon. Optionally, the tissue markers are radio-opaque markers. Optionally, the tissue markers are made of gold, platinum or tantalum. Optionally, the axis of the marker support lies substantially at the center of the polygon.

In the description and claims of the present application, each of the verbs, "comprise," "include" and "have," and conjugates thereof, are used to indicate that the object or objects of the verb are not necessarily a complete listing of components, elements or parts of the subject or subjects of the verb.

Descriptions of embodiments of the invention in the present application are provided by way of example and are not intended to limit the scope of the invention. The described embodiments comprise different features, not all of which are required in all embodiments of the invention. Some embodiments utilize only some of the features or possible combinations of the features. Variations of embodiments of the invention that are described, and embodiments of the invention comprising different combinations of features noted in the described embodiments, will occur to persons of the art. The scope of the invention is limited only by the claims.

## Claims

1. An apparatus (10) for determining a location of a surface of an internal organ of a body, the apparatus comprising:
a marker support (12) having proximal and distal ends, an axis, a collapsed state and a deployed state, the marker support comprising:
at least three flexible filaments, which in the collapsed state lie within a relatively small radial distance from the axis of the marker support, and which upon deployment, each filament forms a loop, the loops each configured to abut a surface of an internal body tissue at an abutment region (27) of each filament,
at least three tissue markers (28), each tissue marker located at an abutment region of a filament
**characterized in that**, the loops are configured, upon moving the proximal and distal ends closer to each other, to then rotate from a radial position to a position in which a vector that extends radially from the axis and intersects the abutment region is substantially normal to a plane in which the abutment region lies.

2. The apparatus according to claim 1 wherein upon initial transition from the collapsed state to the deployed state, the loops define planes which extend along radial directions from the axis of the marker support.

3. The apparatus according to claim 1 or 2 wherein the marker support further comprises a proximal collar (16) at a proximal end and a distal collar (14) at a distal end, each filament connected to the proximal collar and distal collar.

4. The apparatus according to claim 3 wherein bringing the proximal collar closer to the distal collar transforms the marker support from a collapsed state to a deployed state.

5. The apparatus according to any of the previous claims wherein in the deployed state, the tissue markers are configured to abut a surface of an internal body tissue by movement of the marker support in a proximal direction.

6. The apparatus according to any of the previous claims wherein a tissue marker is located substantially midway between the distal and proximal ends of the filament.

7. The apparatus according to any of the previous claims, when the marker support is in a collapsed state, the radial diameter is small enough so that the marker support may be introduced into a human body using a guiding catheter system having dimensions adapted for introduction through the cardiovascular system and passage through the aortic valve.

8. The apparatus according to claim 3 or 4 further comprising a guidewire (30) and a deployment tube (20) external to the guidewirewherein the marker support is slidably attached to a guidewire.

9. The apparatus according to claim 8 wherein the marker support is fixedly attached to the deployment tube at the proximal end of the marker support.

10. The apparatus according to claim 8 or 9 further comprising a connector (26) fixedly attached to the guidewire, the connector configured to engage the distal collar of the marker support upon relative motion of the connector in the direction of the distal collar.

11. The apparatus according to claim 10 wherein a protrusion (34, 44) on the connector engages the distal collar of the marker support, preventing rotational movement of the distal collar relative to the connector.

12. The apparatus according to claim 10 or 11 wherein the connector and distal collar interlock via a clipping mechanism located on the connector (34) and the distal collar (36).

13. The apparatus according to claim 8 further comprising an intermediate tube between the guidewire and the deployment tube wherein the distal collar is fixedly attached to the intermediate tube and the proximal collar is fixedly attached to the deployment tube.

14. An apparatus according to any of the previous claims wherein the marker support comprises a distal marker (24) on its distal end and a proximal marker (18) on its proximal end configured to be imaged overlapping or at a predetermined distance from each other when the marker support is in its deployed state.

15. The apparatus according to any of the previous claims wherein the marker support comprises a superelastic alloy.

## Patentansprüche

1. Vorrichtung (10) zum Bestimmen einer Position einer Fläche eines inneren Organs eines Körpers, wobei die Vorrichtung Folgendes umfasst:
einen **Marker-Träger** (12) mit proximalen und distalen Enden, einer Achse, einen eingefahrenen Zustand und einem ausgefahrenen Zustand, wobei der Marker-Träger Folgendes umfasst:
wenigstens drei flexible **Filamente,** die im eingefahrenen Zustand einen relativ geringen radialen Abstand von der Achse des Marker-Trägers aufweisen, wobei beim Ausfahren jedes Filament eine **Schlaufe** bildet, wobei die Schlaufen jeweils derart konfiguriert sind, dass sie an einer Anlageregion (27) jedes Filaments an einer Fläche eines inneren Körpergewebes anliegen,
wenigstens drei **Gewebemarker (28),** wobei jeder Gewebemarker an einer Anlageregion eines Filaments angeordnet ist,
**dadurch gekennzeichnet, dass** die Schlaufen dazu konfiguriert sind, sich beim Bewegen des proximalen und distalen Endes näher aneinander heran aus einer radialen Position in eine Position zu drehen, in der ein Vektor, der sich radial von der Achse erstreckt und die Anlageregion schneidet, im Wesentlichen senkrecht zu einer Ebene ist, in der die Anlageregion liegt.

2. Vorrichtung nach Anspruch 1, wobei die Schlaufen beim ersten Übergang vom eingefahrenen Zustand zum ausgefahrenen Zustand Ebenen definieren, die sich in radialen Richtungen von der Achse des Marker-Trägers erstrecken.

3. Vorrichtung nach Anspruch 1 oder 2, wobei der Marker-Träger ferner einen proximalen Bund (16) an einem proximalen Ende und einen distalen Bund (14) an einem distalen Ende umfasst, wobei jedes Filament mit dem proximalen Bund und dem distalen Bund verbunden ist.

4. Vorrichtung nach Anspruch 3, wobei der Marker-Träger aus einem eingefahrenen Zustand in einen ausgefahrenen Zustand umgewandelt wird, indem der proximale Bund näher an den distalen Bund gebracht wird.

5. Vorrichtung nach einem der vorangehenden Ansprüche, wobei die Gewebemarker dazu konfiguriert sind, im ausgefahrenen Zustand an einer Fläche eines inneren Körpergewebes anzulegen, indem der Marker-Träger in eine proximale Richtung bewegt wird.

6. Vorrichtung nach einem der vorangehenden Ansprüche, wobei ein Gewebemarker im Wesentlichen in der Mitte zwischen dem distalen und dem proximalen Ende des Filaments angeordnet ist.

7. Vorrichtung nach einem der vorangehenden Ansprüche, wobei, wenn der Marker-Träger in einem eingefahrenen Zustand ist, der radiale Durchmesser klein genug ist, damit der Marker-Träger mithilfe eines Führungskathetersystems mit Abmessungen, die zum Einführen durch das kardiovaskuläre System und das Hindurchtreten durch die Aortenklappe angepasst sind, in einen menschlichen Körper eingeführt werden kann.

8. Vorrichtung nach Anspruch 3 oder 4, ferner umfassend einen Führungsdraht (30) und ein Ausfahrrohr (20) außerhalb des Führungsdrahts, wobei der Marker-Träger verschiebbar an einem Führungsdraht angebracht ist.

9. Vorrichtung nach Anspruch 8, wobei der Marker-Träger am proximalen Ende des Marker-Trägers fest an dem Ausfahrrohr angebracht ist.

10. Vorrichtung nach Anspruch 8 oder 9, ferner umfassend einen Verbinder (26), der fest am Führungsdraht angebracht ist, wobei der Verbinder dazu konfiguriert ist, bei einer relativen Bewegung des Verbinders in Richtung des distalen Bundes in Eingriff mit dem distalen Bund des Marker-Trägers zu treten.

11. Vorrichtung nach Anspruch 10, wobei ein Vorsprung (34, 44) am Verbinder in Eingriff mit dem distalen Bund des Marker-Trägers tritt, um eine Drehbewegung des distalen Bundes relativ zum Verbinder zu verhindern.

12. Vorrichtung nach Anspruch 10 oder 11, wobei der Verbinder und der distale Bund mittels eines Klemmmechanismus, der an dem Verbinder (34) und dem distalen Bund (36) angeordnet ist, ineinander einrasten.

13. Vorrichtung nach Anspruch 8, ferner umfassend ein intermediäres Rohr zwischen dem Führungsdraht und dem Ausfahrrohr, wobei der distale Bund fest an dem intermediären Rohr angebracht ist und der proximale Bund fest am Ausfahrrohr angebracht ist.

14. Vorrichtung nach einem der vorangehenden Ansprüche, wobei der Marker-Träger einen distalen Marker (24) an seinem distalen Ende und einen proximalen Marker (18) an seinem proximalen Ende aufweist, die dazu konfiguriert sind, in Überlagerung oder in einem vorgegebenen Abstand voneinander abgebildet zu werden, wenn der Marker-Träger in seinem ausgefahrenen Zustand ist.

15. Vorrichtung nach einem der vorangehenden Ansprüche, wobei der Marker-Träger eine superelastische Legierung umfasst.

## Revendications

1. Appareil (10) servant à déterminer une position d'une surface d'un organe interne d'un corps, l'appareil comprenant :
un **support de marqueurs** (12) ayant des extrémités proximale et distale, un axe, un état replié et un état déployé, le support de marqueurs comprenant :
au moins trois **filaments** flexibles, qui se trouve à l'état replié à une distance radiale relativement faible de l'axe du support de marqueurs et qui forment chacun une boucle lors du déploiement, les **boucles** étant chacune configurées de manière à venir en butée contre une surface d'un tissu corporel interne au niveau d'une région de butée (27) de chaque filament,
au moins trois **marqueurs de tissu (28),** chaque marqueur de tissu étant situé au niveau d'une région de butée d'un filament
**caractérisé en ce que** les boucles sont configurées, lors du rapprochement des extrémités proximale et distale, pour tourner ensuite d'une position radiale dans une position dans laquelle un vecteur, qui s'étend radialement depuis l'axe et qui coupe la région de butée, est sensiblement perpendiculaire à un plan dans lequel se trouve la région de butée.

2. Appareil selon la revendication 1, dans lequel, lors de la transition initiale de l'état replié dans l'état déployé, les boucles définissent des plans qui s'étendent dans des directions radiales depuis l'axe du support de marqueurs.

3. Appareil selon la revendication 1 ou la revendication 2, dans lequel le support de marqueurs comprend en outre un collier proximal (16) à une extrémité proximale et un collier distal (14) à une extrémité distale, chaque filament étant relié au collier proximal et au collier distal.

4. Appareil selon la revendication 3, dans lequel le rapprochement du collier proximal du collier distal fait passer le support de marqueurs d'un état replié dans un état déployé.

5. Appareil selon l'une quelconque des revendications précédentes, dans lequel, à l'état déployé, les marqueurs de tissu sont configurés pour venir en butée contre une surface d'un tissu corporel interne par un mouvement du support de marqueurs dans une direction proximale.

6. Appareil selon l'une quelconque des revendications précédentes, dans lequel un marqueur de tissu est situé sensiblement à mi-chemin entre les extrémités distale et proximale du filament.

7. Appareil selon l'une quelconque des revendications précédentes, dans lequel, lorsque le support de marqueurs se trouve dans un état replié, le diamètre radial est suffisamment faible pour que le support de marqueurs puisse être introduit dans un corps humain à l'aide d'un système de cathéter de guidage ayant des dimensions adaptées pour l'introduction à travers le système cardio-vasculaire et le passage à travers la valve aortique.

8. Appareil selon la revendication 3 ou la revendication 4, comprenant en outre un fil de guidage (30) et un tube de déploiement (20) à l'extérieur du fil de guidage, le support de marqueurs étant fixé de manière coulissante à un fil de guidage.

9. Appareil selon la revendication 8, dans lequel le support de marqueurs est solidaire tube de déploiement à l'extrémité proximale du support de marqueurs.

10. Appareil selon la revendication 8 ou la revendication 9, comprenant en outre un connecteur (26) solidaire du fil de guidage, le connecteur étant configuré pour s'engager avec le collier distal du support de marqueurs lors d'un mouvement relatif du connecteur dans la direction du collier distal.

11. Appareil selon la revendication 10, dans lequel une saillie (34, 44) sur le connecteur s'engage avec le collier distal du support de marqueurs, empêchant le mouvement de rotation du collier distal par rapport au connecteur.

12. Appareil selon la revendication 10 ou la revendication 11, dans lequel le connecteur et le collier distale se verrouille l'un à l'autre par le biais d'un mécanisme d'encliquetage situé sur le connecteur (34) et le collier distal (36).

13. Appareil selon la revendication 8, comprenant en outre un tube intermédiaire entre le fil de guidage et le tube de déploiement, le collier distal étant solidaire du tube intermédiaire et le collier proximal étant solidaire du tube de déploiement.

14. Appareil selon l'une quelconque des revendications précédentes, dans lequel le support de marqueurs comprend un marqueur distal (24) sur son extrémité distale et un marqueur proximal (18) sur son extrémité proximale, configurés pour être représentés à recouvrement ou à une distance prédéterminée l'un de l'autre lorsque le support de marqueurs se trouve dans son état déployé.

15. Appareil selon l'une quelconque des revendications précédentes, dans lequel le support de marqueurs comprend un alliage superélastique.
